(19) Europäisches Patentamt
European Patent Office
Office européen des brevets

(11) Veröffentlichungsnummer: **0 303 206 A2**

# (12) EUROPÄISCHE PATENTANMELDUNG

(21) Anmeldenummer: **88112834.2**

(51) Int. Cl.[4]: **C07D 207/323**

(22) Anmeldetag: **06.08.88**

(30) Priorität: **14.08.87 DE 3727114**

(43) Veröffentlichungstag der Anmeldung:
**15.02.89 Patentblatt 89/07**

(84) Benannte Vertragsstaaten:
**BE DE FR GB IT NL**

(71) Anmelder: **BASF Aktiengesellschaft**
**Carl-Bosch-Strasse 38**
**D-6700 Ludwigshafen(DE)**

(72) Erfinder: **Hoelderich, Wolfgang, Dr.**
**Mannheimer Strasse 18 c**
**D-6710 Frankenthal(DE)**
Erfinder: **Hesse, Michael, Dr.**
**An der Froschlache 3**
**D-6700 Ludwigshafen(DE)**
Erfinder: **Siegel, Hardo, Dr.**
**Hans-Purrmann-Allee 25**
**D-6720 Speyer(DE)**

## (54) Verfahren zur Herstellung von Pyrrolen.

(57) Die Anmeldung betrifft ein Verfahren zur Herstellung von Pyrrolen der Formel I

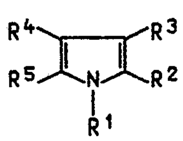

(I),

bei dem man Dialkoxytetrahydrofurane der Formel II

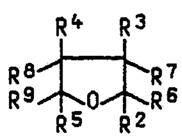

(II),

wobei die Reste $R^2$ bis $R^5$ in den Formeln (I) und (II) einander gleich oder verschieden sind und für Wasserstoff, geradkettige oder verzweigte Alkyl-oder Alkenylreste mit bis zu 12 Kohlenstoffatomen, Cycloalkyl- oder Cycloalkenylreste mit 5 bis 8 Kohlenstoffatomen, Aryl-, Alkylaryl- oder Aralkylreste mit 6 bis 16 Kohlenstoffatomen stehen, und wobei zwei nicht benachbarte Reste von $R^6$ bis $R^9$ für Alkoxyreste und die anderen beiden Reste von $R^6$ bis $R^9$ für Wasserstoff stehen, mit Ammoniak oder primären Aminen $H_2NR^1$, wobei $R^1$ in Formel (I) für Wasserstoff, Alkyl-, Aryl-, Alkylaryl-, Aralkyl- und Cycloalkylreste steht, in Gegenwart von sauren, festen Heterogenkatalysatoren umsetzt. Als feste Heterogenkatalysatoren kann man Zeolithe z.B. des Pentasiltyps, Faujasittyps oder Phosphate, saure Oxide, Phosporsäure oder Borsäure auf Trägern verwenden.

EP 0 303 206 A2

## Verfahren zur Herstellung von Pyrrolen

Die Erfindung betrifft ein Verfahren zur Herstellung von Pyrrolen durch Umsetzung von Dialkoxytetrahydrofuranen mit Ammoniak bzw. primären Aminen in Gegenwart von sauren, festen Heterogenkatalysatoren.

Für die Synthese von Pyrrolen existieren vielfältige Möglichkeiten (J.M. Patterson, Synthesis 281 (1976); E. Baltazzi und L.J. Kirmen, Chem. Rev. 63, 511 (1963)). Besondere Bedeutung für die Herstellung C-substituierter Pyrrole haben die Synthesen nach Paal und Knorr, nach Knorr und nach Hantzsch (A.H. Jackson, Compr. Chem. 4, 275 bis 320 (1979)). N-alkyl-substituierte Pyrrole lassen sich durch katalytische Dehydrierung der entsprechenden Pyrrolidine an $Pd/Al_2O_3$-Katalysatoren herstellen (US 3 008 965, EP 67 360). Auch ist die Herstellung von Pyrrolen aus 2-Butendiolen und Ammoniak bzw. primären Aminen in Gegenwart von Trägerkatalysatoren mit Cu, Ag, Zn, Pd, Ni und Co bekannt (EP 125 415).

Weiterhin ist aus Acta Chem. Scan. 6, 667 bis 670 (1952), J. Org. Chem. 27, 2466 bis 2470 (1962) und US 3 980 089 bekannt, daß einige Dimethoxytetrahydrofurane mit bestimmten primären Aminen in Pyrrole umgewandelt werden, wobei die Reaktion in Essigsäure durchgeführt werden muß.

Es wurde nun gefunden, daß man Pyrrole der Formel I

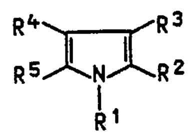

(I),

erhält, wenn man Dialkoxytetrahydrofurane der Formel II

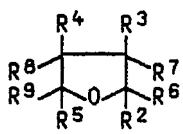

(II),

wobei die Reste $R^2$ bis $R^5$ in den Formeln (I) und (II) einander gleich oder verschieden sind und für Wasserstoff, geradkettige oder verzweigte Alkyl-oder Alkenylreste mit bis zu 12 Kohlenstoffatomen, Cycloalkyl- oder Cycloalkenylreste mit 5 bis 8 Kohlenstoffatomen, Aryl-, Alkylaryl- oder Aralkylreste mit 6 bis 16 Kohlenstoffatomen stehen, und wobei zwei nicht be nachbarte Reste von $R^6$ bis $R^9$ für Alkoxyreste und die anderen beiden Reste von $R^6$ bis $R^9$ für Wasserstoff stehen, mit Ammoniak oder primären Aminen $H_2NR^1$, wobei $R^1$ in Formel (I) für Wasserstoff, Alkyl-, Aryl-, Alkylaryl-, Aralkyl- und Cycloalkylreste steht, in Gegenwart von sauren, festen Heterogenkatalysatoren umsetzt.

Im Vergleich zum Stand der Technik liefert das neue Verfahren auf einfachem und wirtschaftlichem Wege Pyrrole in guter Ausbeute und Reinheit und in guter Raum-Zeit-Ausbeute. Auch die benötigten Katalysatormengen sind geringer. Das erfindungsgemäße Verfahren eignet sich insbesondere für die kontinuierliche Betriebsweise im technischen Maßstab. Alle genannten Vorteile sind überraschend, z.B. daß man bei der erfindungsgemäßen Reaktion in der Gasphase hohe Selektivitäten und Ausbeuten erzielt, da bei den hohen Temperaturen mit Polymerbildung der Pyrrole zu rechnen war. Es ergeben sich auch keine Korrosionsprobleme durch Essigsäure.

Als Reste $R^2$ bis $R^5$ kommen unabhängig von $R^1$ Wasserstoff sowie geradkettige oder verzweigte Alkyl- oder Alkenylreste mit 1 bis 12, insbesondere 1 bis 8, vorzugsweise 1 bis 4 Kohlenstoffatomen in Betracht.

Alkylreste sind z.B. Methyl-, Ethyl-, Propyl-, n-Butyl-, i-Butyl-, Pentyl-, Hexyl-, Octyl- oder Decylreste. Alkenylreste sind z.B. Propenyl-, Butenyl-, Hexenyl- oder Octenylreste.

Als Cycloalkylreste für $R^2$ bis $R^5$ kommen z.B. Cyclopentyl-, Cyclohexyl-, Cycloheptyl-, Cyclopentenyl- oder Cyclohexenylreste in Betracht.

Aromatische Reste für $R^2$ bis $R^5$ sind z.B. Phenyl-, Benzyl-, Toluyl-, Phenylethyl-, p-Methylbenzyl- oder p-Propylphenylreste.

Als Reste $R^6$ bis $R^9$ sind z.B. Methoxy-, Ethoxy-, n-/i-Propoxy-, n-/i-/t-Butoxy-, Hexoxyreste geeignet, wobei aber zwei nicht benachbarte Reste von $R^6$ bis $R^9$ für Wasserstoff stehen.

Die Herstellung der Ausgangsstoffe der Formel (II) ist z.B. in DE-2 710 420 beschrieben.

Neben Ammoniak eignen sich zur Herstellung der entsprechenden Pyrrole der Formel (I) unzersetzt verdampfbare, primäre Amine der Formel (III), beispielsweise Methylamin, Ethylamin, n-Propylamin, i-Propylamin, n-Butylamin, i-Butylamin, s-Butylamin, n-Pentylamin, 3-Methyl-1-butylamin, 3-Methyl-2-butylamin, 2-Methyl-2-butylamin, n-Hexylamin, n-Octylamin, 2-Ethyl-1-hexylamin, Cyclohexylamin, Benzylamin, Anilin, 2-Phenylethylamin, Toluidin.

Beispiele für die aus den Ausgangsstoffen (II) und (III) herstellbaren Endprodukte (I) sind Pyrrol, 1-Methylpyrrol, 1-Ethylpyrrol, 1-n-Propylpyrrol, 1-i-Propylpyrrol, 1-n-Butylpyrrol, 1-i-Butylpyrrol, 1-s-Butylpyrrol, 1-n-Pentylpyrrol, 1-(3-Methyl-1-butyl)-pyrrol, 1-(3-Methyl-2-butyl-pyrrol, 1-(2-Methyl-2-butyl)-pyrrol, 1-n-Hexylpyrrol, 1-n-Octylpyrrol, 1-(2-Ethyl-1-hexyl)-pyrrol, 1-Cyclohexylpyrrol, 1-Benzylpyrrol, 1-Phenylpyrrol, 1-(2-Phenylethyl)-pyrrol, 3-Methylpyrrol, 3,4-Dimethylpyrrol, 2-Methylpyrrol, 2,4-Dimethylpyrrol, 2,5-Dimethylpyrrol, 1,2-Dimethylpyrrol, 1,3-Dimethylpyrrol, 1,2,4-Trimethylpyrrol, 1,3,4-Trimethylpyrrol, 1,2,5-Trimethylpyrrol, 2-Ethypyrrol, 1-Methyl-2-ethylpyrrol, 2-Phenylpyrrol, 1-Methyl-2-phenylpyrrol, 2-Benzylpyrrol, 1-Methyl-2-benzylpyrrol, 2-Cyclohexylpyrrol, 2-(p-Tolyl)-pyrrol.

Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, BAnd 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Aufl., Bd. 24, S. 575 (1983)). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooctaeder, wodurch untersciedlich große Hohlräume und Poren entstehen, unterscheidet man in Zeolithe von Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Type oder Zeolithe vom Faujasit-Typ, z.B. Y-, X- oder L-Zeolithe.

In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et. al. Elsevier Scientific Publishing Comp., 1980, S. 2103 und "Crystal Structures of Ultra-stable Faujasites" Advances in Chemistry Series Nr. 101, American Chemical Society Washington, DC, S. 226ff (1971) und in US-PS 4 512 961.

Besonders geeignet sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengröße, die zwischen denen der Zeolithe vom Type A und denen vom Typ X oder Y liegen (vgl. Ullmanns Encyclopädie d. techn. Chem., 4. Aufl., Bd. 24, 1983).

Diese Zeolithe können unterschiedliche Zusammensetzung aufweisen. Es handelt sich hierbei um Alumino-, Boro-, Eisen-, Beryllium-, Gallium-, Chrom-, Arsen-, Antimon- und Wismutsilikatzeolith oder deren Gemische sowie Alumino-, Boro-, Gallium- und Eisengermanatzeolithe oder deren Gemische. Insbesondere eignen sich Alumino-, Boro- und Eisensilikatzeolithe des Pentasiltyps für das erfindungsgemäße Verfahren. Der Aluminosilikatzelith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Ausgangsstoffmengen ein $SiO_2/Al_2O_3$-Verhältnis von 10 bis 40.000. Man kann diese Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können auch hergestellt werden, wenn man die Reaktion statt in wäßriger Aminlösung in etherischer Lösung, z.B.

Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren silicumreichen Zeolithen ($SiO_2/Al_2O_3 \geq 10$) gehören auch die ZSM-Typen, Ferrierit, NU-1 und Silicalit®, ein Molekularsieb, ein sog. Silica Polymorph.

Die nach diesem Verfahren hergestellten Alumino-, Boro- und Eisensilikatzeolithe können nach ihrer Isolierung, Trocknung bei 100 bis 160 °C, z.B. bei 110 °C und Calcinierung bei 450 bis 550 °C, z.B. bei 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, insbesondere 75:25, Siliciumdioxid, insbesondere hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch gut geeignete Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der Umsetzung bei den zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, insesondere 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalystoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo; W, Mn, Re, Fe, Ru, Os, Co, Rh, Sr, Ni, Pd, Pt, Übergangsmetalle der 1. und 2 Nebengruppe wie Cu, Ag, Zn, Seltene Erdmetalle wie La, Ce, Pr, Nd, Fr, Yb und U eingesetzt.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der genannten Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrig $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt

und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei kann man z.B. so vorgehen, daß man die Zeolithe in Pulverform zuerst mit 1 n Phosphorsäure 1 Stunde bei 80 °C behandelt. Nach dieser Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Verformung bei erhöhter Temperatur mit 0,001 n bis 2 n, vorzugsweise 0,05 n bis 0,5 n Flußsäure behandelt, z.B. durch Erhitzen unter Rückfluß über einen Zetraum von 0,5 bis 5, vorzugsweise 1 bis 3 Stunden. Nach Isolierung, z.B. durch Abfiltrieren und Auswaschen, des zeolithischen Materials wird dieses zweckmäßig, bei Temperaturen von 100 bis 160 °C, getrocknet und bei Temperaturen von 450 °C bis 600 °C calciniert. Gemäß einer anderen Ausführungsform für die Säurebehandlung wird das zeolithische Material nach einer Verformung mit Bindemittel bei Temperaturen von 50 °C bis 90 °C, insbesondere 60 °C bis 80 °C, über einen Zeitraum von 0,5 bis 5, mit 12 bis 20 gew.%iger Salzsäure, behandelt. Anschließend wird das zeolithische Material ausgewaschen und bei Temperaturen von 100 bis 160 °C, getrocknet und bei Temperaturen von 450 bis 600 °C calciniert. Einer HF-Behandlung kann sich auch eine HCl-Behandlung anschließen.

Nach einer anderen Arbeitsweise kann man die Zeolithe durch Aufbringen von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger $H_3PO_4$-Lösung getränkt, bei 110 °C getrocknet und bei 500 °C calciniert.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische. Als Aluminiumphosphat-Katalysatoren werden insbesondere unter hydrothermalen Bedingungen synthetisierte Aluminiumphosphate eingesetzt, die Zeolithstruktur besitzen. Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

$AlPO_4$-5 (APO-5) kann man synthetisieren, indem man Orthophosphorsäure mit Pseudoboehmit (Catapal SB®) in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert.

$AlPO_4$-9 (APO-9) kann gleichfalls aus Orthophosphorsäure und Pseudoboehmit aber in wäßriger DABCO-Lösung (1,4-Diazabicyclo-(2,2,2)octan) bei etwa 200 °C unter autogenem Druck während 200 bis 400 h synthetisiert werden.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Die für das erfindungsgemäße Verfahren eingesetzten Siliciumaluminiumphosphate sind z.B. SAPO-5, SAPO-11, SAPO-31 ud SAPO-34. Die Synthese dieser Verbindungen ist in EP 103 117 und US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Phosphatkatalysatoren kann man bei dem Verfahren auch gefällte Aluminiumphosphate einsetzen. Für die Herstellung eines derartigen Aluminiumphosphates werden z.B. 92 g Diammoniumhydrogenphosphat in 700 ml Wasser gelöst. Zu dieser Lösung wird 260 g $Al(NO_3)_3$ x $H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird

bei 60 °C/16 h getrocknet.

Borphosphate kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, insbesondere 300 bis 500 °C herstellen.

Auf diese Phosphate können durch Imprägnierung (Tränken und Aufsprühen) oder in manchen Fällen auch durch Ionenaustausch Modifizierungskomponenten, wie bei den Zeolithen beschrieben, aufgebracht werden. Auch kann wie bei den Zeolithkatalysatoren eine Modifizierung mit Säuren erfolgen.

Geeignete saure Katalysatoren sind z.B. die sauer wirkenden Oxide von Elementen der III. und IV. Hauptgruppe sowie IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide. Diese Oxide können auch durch Aufbringen von Modifizierungskomponenten, wie bei den Zeolithkatalysatoren beschrieben, dotiert werden. Die Behandlung mit Säuren, wie bei den Zeolithkatalysatoren beschrieben, ist ebenfalls eine Möglichkeit der Modifizierung.

Auch mit Phosphorsäure oder Borsäure getränkte Katalysatoren können eingesetzt werden. Phosphorsäure oder Borsäure wird z.B. auf $SiO_2$-, $Al_2O_3$-, $TiO_2$- oder Bimsträger, z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phophorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die Umwandlung wird vorzugsweise in der Gasphase bei Temperaturen von 100 bis 500 °C, insbesondere 200 bis 400 °C und einer Belastung WHSV = 0,1 bis 20 $h^{-1}$, insbesondere 0,5 bis 5 $h_{-1}$ (g Ausgangsstoff/g Katalysator und Stunde). Das molare Verhältnis zwischen Edukt und Ammoniak bzw. Amin beträgt 1:0,5 bis 1:20, bevorzugt 1:1 bis 1:5. Die Reaktion läßt sich in einem Festbett oder auch Wirbelbett ausführen.

Es ist auch möglich, die Reaktion in der Flüssigphase (Suspension-, Riesel- oder Sumpffahrweise) bei Temperaturen zwischen 50 und 200 °C durchzuführen.

Das Verfahren wird in der Regel bei Normaldruck oder auch je nach Flüchtigkeit der Ausgangsverbindung und Produkte bei vermindertem oder erhöhtem Druck durchgeführt, wobei die Durchführung diskontinuierlich, vorzugsweise kontinuierlich erfolgen kann. Die Reaktionsführung läßt sich auch unter vermindertem Druck gut bewerkstelligen.

Schwerflüchtige oder feste Ausgangsstoffe werden in gelöster Form z.B. in THF-, Toluol- oder Petrolether-Lösung eingesetzt. Generell ist eine Verdünnung des Ausgangsstoffes mit derartigen Lösungsmitteln oder mit Inertgasen wie $N_2$, Ar, $H_2O$-Dampf möglich. In besonderen Fällen kann auch $O_2$ verwendet werden.

Nach der Umsetzung werden die entstandenen Produkte durch übliche Verfahren z.B. durch Destillation aus dem Reaktionsgemisch isoliert; nichtumgesetzte Ausgangsstoffe werden gegebenenfalls in die Umsetzung zurückgeführt.

Vorteilhaft werden die gasförmigen Reaktionsprodukte sofort in eine Trennung, z.B. in eine Fraktionierkolonne, eingebracht und in ihre Einzelkomponenten zerlegt.

Die nach dem erfindungsgemäßen Verfahren hergestellten Pyrrole sind wertvolle Ausgangsstoffe für die Herstellung von Farbstoffen, Korrosionsinhibitoren, Pharmazeutica und Schädlingsbekämpfungsmittel.

Beispiele 1 bis 28

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosiikatzeolith setzt sich zusammen aus 94,2 Gew.% $SiO_2$ und 2,3 Gew.% $B_2O_3$.

Mit diesem Material werden durch Verformen mit einem Verformungshilfsmittel 2-mm-Stränge hergestellt, die bei 110 °C/16 h getrocknet und bei 500 °C/24 h calciniert werden.

Katalysator B

Katalysator B erhält man, indem man den Borosilikatzeolith von Katalysator A, mit Boehmit im Gewichtsverhältnis 60:40 zu 2-mm-Strängen verformt, bei 110 °C/16 h trocknet und bei 500 °C/24 h calciniert.

Katalysator C

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminiumzeolith enthält 91,6 Gew.% $SiO_2$ und 4,6 Gew.% $Al_2O_3$.

Der Katalysator wird mit einem Verformungshilfsmittel zu 2-mm-Strängen verformt, bei 110 °C/16 h getrocknet und bei 500 °C/24 h calciniert.

Katalysator D

Katalyator D erhält man, indem man Katalysator B mit einer wäßrigen $La(NO_3)_3$-Lösung imprägniert, danach bei 130 °C/2 h trocknet und bei 540 °C/2 h calciniert. Der La-Gehalt beträgt 3,2 Gew.%.

Katalysator E

Katalysator E erhält man, indem man die Stränge des Katalysators B mit einer wäßrigen Lösung aus Cernitrat imprägniert, danach bei 130 °C/2 h trocknet und bei 540 °C/2 h calciniert. Der Ce-Gehalt beträgt 2,5 Gew.%.

Katalysator F

Der Eisensilikatzeolith des Pentasil-Tys wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Der Katalysator wird mit hochdispersem $SiO_2$ im Gewichtsverhältnis 80:20 zu 2,5-mm-Strängen verstrangt, bei 110 °C/16 h getrocknet und bei 500 °C/24 calciniert.

Katalysator G

660 g Kieselsol (30 Gew.% $SiO_2$) werden mit 567 g wäßriger Tetrapropylammoniumhydroxid-Lösung (20 %ig) gemischt und in einem Autoklaven bei 200 °C in 72 h umgesetzt. Nach Abtrennen von der Mutterlauge wird bei 120 °C getrocknet und bei 500 °C/16 h calciniert. Das Diffraktogramm zeigt die für Silicalit® typischen Röntgenbeugungsmuster. Dieser Silicalit wird mit hochdispersem $SiO_2$ im Gewichtsverhältnis 70:30 zu 2-mm-Strängen verformt. Diese Stränge werden mit einer 20 %igen $NH_4Cl$-Lösung bei 80 °C in einer Kolonne ionenausgetauscht. Danach wird mit Wasser ausgewaschen, bei 110 °C getrocknet und bei 500 °C/5 h calciniert. Der Na-Gehalt des Silicaliten beträgt nach dieser Verfahrensweise 0,015 Gew.%. Der Ionenaustausch kann wiederholt werden nach Zwischencalcination, um diesen Na-Gehalt einzustellen.

Katalysator H

Katalysator H wird wie Katalysator E hergestellt, wobei jedoch Katalysator C mit $Ce(NO_3)_3$ imprägniert wird. Der Ce-Gehalt beträgt 3,4 Gew.%.

Katalysator I

100 g des bei Katalysator A verwendeten Borosiikatzeolithen werden mit 280 ml einer 0,1 n HF bei 90 °C 2 h lang behandelt und nach Abfiltrieren bei 160 °C getrocknet. Dieses Produkt wird mit amorphem Aluminosilikat (25 Gew.% $Al_2O_3$ und 75 Gew.% $SiO_2$) im Gewichtsverhältnis 60:40 zu 2-mm-Strängen verformt, bei 110 °C/16 h getrocknet und bei 500 °C/16 h calciniert.

Katalysator J

Die Synthese von $AlPO_4$-5 (APO-5) erfolgt durch Zusammenrühren von 200 g 95 %iger Phosphorsäure gelöst in 325 g $H_2O$, 136 g Boehmit und 678 g Tetrapropylammoniumhydroxid (30 %ig) und anschließende Reaktion bei 150 °C unter autogenem Druck während 43 Stunden. Das bei 120 °C getrocknete und bei 500 °C/16 h calcinierte Produkt enthält 46,5 Gew.% $P_2O_5$ und 45,5 Gew.% $Al_2O_3$. Dieses $AlPO_4$-5 wird mit Boehmit im Gewichtsverhältnis 60:40 zu 2-mm-Strängen verformt, bei 110 °C getrocknet und bei 500 °C/16 h calciniert.

Katalysator K

Siliciumaluminiumphosphat-5 (SAPO-5) wird hergestellt aus einer Mischung aus 200 g 98 %iger Phosphorsäure, 136 g Boehmit, 60 g Kieselsol (30 %ig) 287 g Tripropylamin und 587 g $H_2O$. Diese Mischung wird bei 150 °C während 168 Stunden unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$, SAPO-5 wird mit einem Verstrangungshilfsmittel zu 3-mm-Strängen verformt, bei 120 °C getrocknet und bei 500 °C calciniert.

Katalysator I

Kommerziell erhältliches Zirkonphosphat $Zr_3(PO_4)_4$ in Reinsubstanz verformt.

Katalysator M

$CePO_4$ wird durch Fällung aus 52 g $Ce(NO_3)_3 \times 6\ H_2O$ und 56 g $NaH_2PO_4 \times 2\ H_2O$ erhalten. Nach der Filtration wird das Material zu Strängen verformt, bei 120 °C getrocknet und bei 450 °C calciniert. Katalysator I enthält 47,1 Gew.% Ce und 12,7 Gew.% P.

Katalysator N

$BPO_4$ wird hergestellt, indem man 49 g $H_3BO_3$ mit 117 g $H_3PO_4$ (75 %ig) in einem Kneter zusammengibt, überschüssiges Wasser verdampft und das Umsetzungsprodukt zu 3-mm-Strängen verformt. Diese Stränge werden bei 100° C getrocknet und bei 350° C calciniert. Katalysator N enthält 8,77 Gew.% B und 28,3 Gew.% P.

Katalysator O

Katalysator O ist ein gefälltes Aluminiumphosphat, das durch Fällung aus $Al(NO_3)_3$-$H_3PO_4$-Lösung mit $NH_3$ bei pH = 6 bis 7 erhalten wird. Nach Abfiltrieren des Niederschlags wird bei 110° C getrocknet und bei 500° C calciniert. Katalysator O enthält 28,5 Gew.% Al und 13,2 Gew.% P.

Katalysator P

Kommerziell erhältlicher NaY-Zeolith wird mit Boehmit im Gewichtsverhältnis 60:40 zu 2 mm verstrangt, bei 110° C/16 h getrocknet und bei 540° C/24 h calciniert und mit 20 %iger wäßriger ($La(NO_3)_2$-Lösung einem Ionenaustausch bei 80° C/2 h unterworfen. Nach Trocknung bei 110° C und Calcination bei 500° C soll der La-Gehalt 7,1 Gew.% und der Na-Gehalt 1,1 Gew.% betragen. Der Ionenaustausch kann nach Zwischencalcination wiederholt werden bis obige Werte erreicht sind.

Katalysator Q

$SiO_2$ im Handel erhältlich unter D 11-10®.

Katalysator R

$TiO_2$P 25® wird zu 2-mm-Strängen verformt, bei 110° C getrocknet und bei 500° C/16 h calciniert.

Katalysator S

Kieselgel wird mit 85 %iger $H_3PO_4$ und $H_3BO_3$ im Sprühturm behandelt. Das Sprühpulver wird zu Tabletten verformt, bei 120° C getrocknet und bei 500° C/16 h calciniert. Katalysator S enthält 85,9 Gew.% $SiO_2$, 1,56 Gew.% P und 0,06 Gew.% B.

Katalysator T

$Al_2O_3$ im Handel erhältlich unter D 10-10®.

Katalysator U

D 10-10 wird mit $H_3BO_3$ imprägniert, getrocknet bei 110° C und bei 550° C/5 h calciniert. Der Katalysator U setzt sich zusammen aus 85 % $Al_2O_3$ und 15 % $B_2O_3$.

Katalysator V

Katalysator V wird erhalten, indem man D 10-10 $Al_2O_3$ mit 85 %iger $H_3PO_4$ 30 min behandelt, danach 130° C/2 h getrocknet und bei 540° C/2 h calciniert. Der P-Gehalt beträgt 4,9 Gew.%.

Katalysator W

200 g Katalysator R werden mit 600 ml 15 %iger HCl bei 80° C/1 h behandelt. Danach wird das Material Cl-frei gewaschen, bei 110° C getrocnet und bei 600° C/1 h calciniert.

Die mit diesen Katalysatoren erzielten Versuchsergebnisse und Versuchsbedingungen sind in Tabelle 1 bis Tabelle 3 zusammengefaßt.

Man erkennt, daß die zeolithischen Katalysatoren unter genannten Katalysatoren am besten für das erfindungsgemäße Verfahren geeignet sind.

Tabelle 1

| 1,4-Dimethoxytetrahydrofuran (I) + $NH_3$→Pyrrol (II) + $H_2O$ + 2 $CH_3OH$ | | | | | |
|---|---|---|---|---|---|
| Beispiel | 1 | 2 | 3 | 4 | 5 |
| Katalysator | A | C | F | I | G |
| Temperatur °C | 400 | 400 | 400 | 350 | 400 |
| WHSV $h^{-1}$ | 3 | 3 | 3 | 3 | 3 |
| Molverhältnis I:$NH_3$ | 1 : 3 | 1 : 3 | 1 : 3 | 1 : 3 | 1 : 3 |
| Umsatz (I) | 100 | 100 | 100 | 98,8 | 100 |
| Selektivität (II) | 85,7 | 80,5 | 79,0 | 84,5 | 68,4 |

Tabelle 2

| 1,4 - Dimethoxytetrahydrofuran (I) + $NH_3$→Pyrrol (II) + $H_2O$ + 2 $CH_3OH$ | | | | | | | | | |
|---|---|---|---|---|---|---|---|---|---|
| Beispiel | 6 | 7 | 8 | 9 | 10 | 11 | 12 | 13 | 14 |
| Katalysator | B | B | D | E | H | I | K | L | M |
| Temperatur °C | 300 | 350 | 350 | 350 | 350 | 400 | 400 | 400 | 400 |
| WHSV $h^{-1}$ | 3 | 3 | 3 | 3 | 3 | 3 | 3 | 3 | 3 |
| Molverhältnis I:$NW_3$ | 1 : 3 | 1 : 3 | 1 : 3 | 1 : 3 | 1 : 3 | 1 : 3 | 1 : 3 | 1 : 3 | 1 : 3 |
| Zusammensetzung des flüssigen Produktgemisches in FL % | | | | | | | | | |
| I | 26,7 | - | - | - | - | 13,8 | 1,5 | 36,9 | 37,6 |
| II | 47,8 | 63,9 | 67,4 | 64,3 | 63,9 | 42,4 | 69,4 | 25,3 | 31,8 |
| $CH_3OH$ | 18,1 | 22,1 | 24,1 | 21,3 | 21,9 | 21,8 | 24,8 | 15,8 | 13,0 |
| Mithoxydihydrofuran | 2,7 | 5,6 | 4,2 | 7,1 | 5,9 | 5,1 | 1,8 | 9,4 | 5,0 |
| N-Methylpyrrol | 1,9 | - | 1,7 | 2,5 | - | 0,7 | 0,8 | 0,2 | 2,6 |
| Rest* | 2,8 | 8,4 | 2,6 | 4,8 | 8,3 | 16,2 | 1,7 | 12,4 | 10,0 |

*nicht näher identifiziert

Forsetzung Tabelle 2

| Beispiel | 15 | 16 | 17 | 18 | 19 | 20 | 21 | 22 | 23 | 24 |
|---|---|---|---|---|---|---|---|---|---|---|
| Katalysator | N | O | P | Q | R | S | T | U | V | W |
| Temperatur °C | 400 | 400 | 350 | 400 | 400 | 400 | 400 | 400 | 400 | 400 |
| WHSV $h^{-1}$ | 3 | 3 | 3 | 3 | 3 | 3 | 3 | 3 | 3 | 3 |
| Molverhältnis I:$NW_3$ | 1 : 3 | 1 : 3 | 1 : 3 | 1 : 3 | 1 : 3 | 1 : 3 | 1 : 3 | 1 : 3 | 1 : 3 | 1 : 3 |
| Zusammensetzung des flüssigen Produktgemisches in FL % | | | | | | | | | | |
| I | 30,4 | 8,3 | 21,0 | 0,4 | 52,4 | 0,6 | 4,5 | 4,9 | 14,0 | 59,6 |
| II | 36,2 | 45,5 | 44,1 | 43,3 | 29,7 | 55,1 | 52,7 | 53,5 | 45,6 | 22,3 |
| $CH_3OH$ | 7,7 | 25,3 | 20,6 | 30,4 | 10,5 | 26,6 | 23,3 | 25,1 | 17,5 | 8,8 |
| Mithoxydihydrofuran | 4,7 | 4,6 | 5,2 | 8,9 | 4,6 | 1,4 | 2,2 | 2,7 | 3,0 | 6,0 |
| N-Methylpyrrol | 12,5 | 2,2 | 0,8 | 0,3 | 0,1 | 6,8 | 0,6 | 4,3 | 6,6 | - |
| Rest * | 8,5 | 14,1 | 8,3 | 17,0 | 2,7 | 9,5 | 16,7 | 9,5 | 13,3 | 3,3 |

*nicht näher identifiziert

Tabelle 3

| 2-Methylpyrrol aus 2-Methyl-2,5-dimethoxytetrahydrofuran und $NH_3$ | | | | |
|---|---|---|---|---|
| Beispiel | 25 [1)] | 26 [1)] | 27 [1)] | 28 [1)] |
| Katalysator | B | E | I | C |
| Edukt/$NH_3$ molar | 2,5 | 2,5 | 2,5 | 2,5 |
| Temp., °C | 350 | 350 | 350 | 350 |
| WHSV, $h^{-1}$ | 1,5 | 1,5 | 1,5 | 1,5 |
| Umsatz | 100 | 100 | 100 | 100 |
| Selektivität | 68,9 | 71,4 | 82,3 | 66,5 |

[1)] 2-Methyl-2,5-dimethoxytetrahydrofuran in THF gelöst (50 : 50)

## Ansprüche

1. Verfahren zur Herstellung von Pyrrolen der Formel I

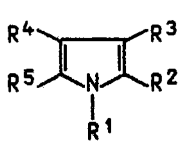

(I),

dadurch gekennzeichnet, daß man Dialkoxytetrahydrofurane der Formel II

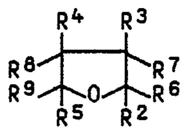

(II),

wobei die Reste $R^2$ bis $R^5$ in den Formeln (I) und (II) einander gleich oder verschieden sind und für Wasserstoff, geradkettige oder verzweigte Alkyl- oder Alkenylreste mit bis zu 12 Kohlenstoffatomen, Cycloalkyl- oder Cycloalkenylreste mit 5 bis 8 Kohlenstoffatomen, Aryl-, Alkylaryl- oder Aralkylreste mit 6 bis 16 Kohlenstoffatomen stehen, und wobei zwei nicht benachbarte Reste von $R^6$ bis $R^9$ für Alkoxyreste und die anderen beiden Reste von $R^6$ bis $R^9$ für Wasserstoff stehen, mit Ammoniak oder primären Aminen $H_2NR^1$, wobei $R^1$ in Formel (I) für Wasserstoff, Alkyl-, Aryl-, Alkylaryl-, Aralkyl- und Cycloalkylreste steht, in Gegenwart von sauren, festen Heterogenkatalysatoren umsetzt.

2. Verfahren nach Anspruch 1, dadurch gekennzeichnet, daß man als Heterogenkatalysatoren Zeolithe verwendet.

3. Verfahren nach Anspruch 1 und 2, dadurch gekennzeichnet, daß man als Heterogenkatalysatoren Zeolithe des Pentasiltyps verwendet.

4. Verfahren nach Anspruch 1 bis 3, dadurch gekennzeichnet, daß man als Katalysatoren Aluminium-, Boro oder Eisensilikatzeolithe des Pentasiltyps verwendet.

5. Verfahren nach Anspruch 1 und 2, dadurch gekennzeichnet, daß man als Katalysatoren Zeolithe des Faujasittyps verwendet.

6. Verfahren nach Anspruch 1 bis 5, dadurch gekennzeichnet, daß man mit Alkalimetallen, Übergangsmetallen, Seltenen Erdmetallen dotierte Zeolithe als Katalysatoren verwendet.

7. Verfahren nach Anspruch 1, dadurch gekennzeichnet, daß man als Heterogenkatalysatoren Phosphate, saure Oxide und/oder Phosphorsäure bzw. Borsäure auf Trägermaterialien verwendet.

8. Verfahren nach Anspruch 1 und 7, dadurch gekennzeichnet, daß man als Heterogenkatalysatoren hydrothermal hergestellte Phosphate verwendet.

9. Verfahren nach Anspruch 1 und 7, dadurch gekennzeichnet, daß man als Heterogenkatalysatoren saure Oxide der Elemente Si, Ti, Zr, B, Fe, W, Mo, Nb, V verwendet.

10. Verfahren nach Anspruch 1 bis 9, dadurch gekennzeichnet, daß man die Reaktion in der Gasphase durchführt.